# EUROPEAN PATENT APPLICATION

(11) **EP 4 458 857 A1**
(43) Date of publication of application: **06.11.2024**
(21) Application number: 24173170.2
(22) Date of filing: 29.04.2024
(51) Int. Cl.: C07K 16/28, C07K 14/705, C07K 14/725, C07K 19/00, A61K 35/17, A61P 35/02, C12N 5/10

(54) **MURINE ANTI-CD19 CHIMERIC ANTIGEN RECEPTOR FOR TREATING CANCER**

(30) Priority: 03.05.2023 US 202318311409
(71) Applicant: Pell Bio-Med Technology Co., Ltd., Taipei City (TW)
(72) Inventor: Lin, Chen-Lung, Taipei City 114 (TW)
(74) Representative: Rimini, Rebecca

(57) **Abstract**

Provided is an isolated nucleic acid molecule encoding a chimeric antigen receptor (CAR), in which the CAR includes a single chain antibody or single chain antibody fragment, a costimulatory domain, a primary intracellular signaling domain. The single chain antibody or single chain antibody fragment includes a murine anti-CD19 binding domain, wherein the murine anti-CD19 binding domain includes a heavy chain variable (VH) region comprising a heavy chain complementarity determining region (CDR H1), a CDR H2, and a CDR H3; and a light chain variable (VL) region comprising a light chain complementarity determining region 1 (CDR L1), a CDR L2, and a CDR L3. The costimulatory domain includes 4-1 BB, and the primary intracellular signaling domain includes a native intracellular signaling domain of CD3 zeta.

## Description

### BACKGROUND

### Field of Invention

The present invention relates to chimeric antigen receptor. More particularly, the present invention relates to murine anti-CD19 chimeric antigen receptor.

### Description of Related Art

Tumor cells have many properties that facilitate immune evasion. Recently, chimeric antigen receptor T cells (CAR-Ts) are designed to specifically target the tumor cells. CAR-Ts are produced by genetically modifying autologous T cells from the patients. Through procedures of isolation, activation, gene-modification, and expansion, pre-designed genes are introduced into the patient T cells, generating CAR-Ts that simultaneously express specific cancer targeting motifs via their CAR structure while exhibiting enhanced cytotoxicity against cancer cells. The completely formulated CAR-Ts are then re-infused into the patient to treat cancer.

Notwithstanding the foregoing efforts, there remains a continuing need for more effective CAR-Ts. Therefore, the related art needs to be improved.

### SUMMARY

The present disclosure provides an isolated nucleic acid molecule encoding a chimeric antigen receptor (CAR), wherein the CAR comprises a single chain antibody or single chain antibody fragment, a costimulatory domain, a primary intracellular signaling domain. The single chain antibody or single chain antibody fragment comprises a murine anti-CD19 binding domain, wherein the murine anti-CD19 binding domain comprises a heavy chain variable (VH) region comprising a heavy chain complementarity determining region (CDR H1), a CDR H2, and a CDR H3; and a light chain variable (VL) region comprising a light chain complementarity determining region 1 (CDR L1), a CDR L2, and a CDR L3. The CDR H1 comprises SEQ ID NO: 1, the CDR H2 comprises SEQ ID NO: 2, and the CDR H3 comprises SEQ ID NO: 3; and the CDR L1 comprises SEQ ID NO: 4, the CDR L2 comprises SEQ ID NO: 5, and the CDR L3 comprises SEQ ID NO: 6. The costimulatory domain comprises 4-1BB, and the primary intracellular signaling domain comprises a native intracellular signaling domain of CD3 zeta.

In some embodiments, the murine anti-CD19 binding domain is a single chain variable fragment (scFv), a di-scFv, a fragment antigen binding (Fab), or F(ab')2.

In some embodiments, the VH region comprises SEQ ID NO: 7 or a sequence with at least 90% identity thereof; and the VL region comprises SEQ ID NO: 8, or a sequence with at least 90% identity thereof.

In some embodiments, the murine anti-CD19 binding domain comprises an amino acid sequence of SEQ ID NO: 9, or an amino acid sequence with at least 90% identity thereof.

In some embodiments, the murine anti-CD19 binding domain comprises a nucleic acid sequence encoding the murine anti-CD19 binding domain, wherein the nucleic acid sequence comprises SEQ ID NO: 10, or the nucleic acid sequence with at least 90% identity thereof.

In some embodiments, the 4-1BB comprises an amino acid sequence of SEQ ID NO: 13, or an amino acid sequence with at least 90% identity thereof.

In some embodiments, the 4-1BB comprises a nucleic acid sequence encoding the 4-1BB, and the nucleic acid sequence comprises SEQ ID NO: 14, or the nucleic acid sequence with at least 90% identity thereof.

In some embodiments, the CD3 zeta comprises an amino acid sequence of SEQ ID NO: 15, or an amino acid sequence with at least 90% identity thereof.

In some embodiments, the CD3 zeta comprises a nucleic acid sequence encoding the CD3 zeta, and the nucleic acid sequence comprises SEQ ID NO: 16, or the nucleic acid sequence with at least 90% identity thereof.

In some embodiments, the CAR sequentially comprising the single chain antibody or single chain antibody fragment, the costimulatory domain, and the primary intracellular signaling domain.

In some embodiments, the isolated nucleic acid molecule encoding the CAR further comprises a transmembrane domain of CD8 hinge.

In some embodiments, the CD8 hinge comprises an amino acid sequence of SEQ ID NO: 11, or an amino acid sequence with at least 90% identity thereof.

In some embodiments, the CD8 hinge comprises a nucleic acid sequence encoding the CD8 hinge, and the nucleic acid sequence comprises SEQ ID NO: 12, or the nucleic acid sequence having at least 90% identity thereof.

In some embodiments, the CAR comprises an amino acid sequence of SEQ ID NO: 17, or an amino acid sequence with at least 90% identity thereof.

In some embodiments, the CAR comprises a nucleic acid sequence encoding the CAR, wherein the nucleic acid sequence comprises SEQ ID NO: 18 or the nucleic acid sequence with at least 90% identity thereof.

The present disclosure also provides a vector comprising the nucleic acid molecule encoding the CAR as above mentioned.

In some embodiments, the vector is selected from the group consisting of a DNA, a RNA, a plasmid, a lentivirus vector, an adenoviral vector, or a retrovirus vector.

In some embodiments, the vector further comprises an EF-1 promoter.

The present disclosure also provides a cell comprising the vector as above mentioned, wherein the cell is a human T cell.

In some embodiments, the T cell is a CD8+ T cell, a CD4+ T cell, or a combination thereof.

### BRIEF DESCRIPTION OF THE DRAWINGS

Aspects of the present disclosure are best understood from the following detailed description when read with the accompanying figures. It is noted that, in accordance with the standard practice in the industry, various features are not drawn to scale. In fact, the dimensions of the various features may be arbitrarily increased or reduced for clarity of discussion. The disclosure can be more fully understood by reading the following detailed description of the embodiment, with reference made to the accompanying drawings as follows:
Fig. 1 is surface CD19 expression on Raji cancer cell line under the P1 gating using flow cytometry.
Fig. 2A is CD107a degranulation of untransduced (UTD) and CAR transduced primary T cells (CD19.BB.z) after 5 hours co-culturing with CD19 Raji cancer cells.
Fig. 2B is CD107a degranulation of untransduced (CD4 UTD, CD8 UTD) and CAR transduced primary T cells including CD4+ CAR-T subset (CD4 CD19.BB.z) and CD8+ CAR-T subset (CD8 CD19.BB.z) after 5 hours co-culturing with CD19 Raji cancer cells.
Fig. 3 is specific cell lysis of Raji cells after 48 hours of co-culturing with untransduced (UTD) and CAR transduced primary T cells (mCD19) measured by luciferase (Luc) based cytotoxicity assay.
Fig. 4 is proliferation of CAR transduced primary T cells without or with Raji cell exposure at different E:T ratio measured by flow cytometry.
Fig. 5 is modeling of the CAR-T proliferation of Fig. 4.
Fig. 6 is a study design for leukemia xenograft animal model.
Fig. 7 is bioluminescent images of mice of G1 to G4 were obtained at indicated periods in the same imaging condition.
Fig. 8 is *in vivo* bioluminescence intensity of organs (heart, liver, spleen, lung, kidney, brain and femur) and whole body of Raji/Luc transplanted mice on the day of sacrifice.
Fig. 9 is Kaplan-Meier curve of Raji/Luc cancer model administrated with CAR-T. Burkitt's lymphoma-free survival curve.

### DETAILED DESCRIPTION

The following disclosure provides detailed description of many different embodiments, or examples, for implementing different features of the provided subject matter. These are, of course, merely examples and are not intended to limit the invention but to illustrate it. In addition, various embodiments disclosed below may combine or substitute one embodiment with another, and may have additional embodiments in addition to those described below in a beneficial way without further description or explanation. In the following description, many specific details are set forth to provide a more thorough understanding of the present disclosure. It will be apparent, however, to those skilled in the art, that the present disclosure may be practiced without these specific details.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising", or "includes" and/or "including" or "has" and/or "having" when used in this specification, specify the presence of stated features, regions, integers, steps, operations, elements, and/or components, but do not preclude the presence or addition of one or more other features, regions, integers, steps, operations, elements, components, and/or groups thereof.

In some embodiments, an isolated nucleic acid molecule encoding a CAR, in which the CAR comprises a single chain antibody or single chain antibody fragment comprising a murine anti-CD19 binding domain, a transmembrane domain comprising CD8 hinge, a costimulatory domain comprising 4-1BB, and a primary intracellular signaling domain comprising a native intracellular signaling domain of CD3 zeta.

In some embodiments, the murine anti-CD19 binding domain comprises a heavy chain variable (VH) region comprising a CDR H1, a CDR H2, and a CDR H3; and a light chain variable (VL) region comprising a CDR L1, a CDR L2, and a CDR L3. The CDR H1 comprises SEQ ID NO: 1, the CDR H2 comprises SEQ ID NO: 2, and the CDR H3 comprises SEQ ID NO: 3; and the CDR L1 comprises SEQ ID NO: 4, the CDR L2 comprises SEQ ID NO: 5, and the CDR L3 comprises SEQ ID NO: 6. The CDR regions are define by Kabat definition.

In some embodiments, a full length of VH region comprises SEQ ID NO: 7 and a full length of VL region comprises SEQ ID NO: 8, or a sequence having at least 90% identity thereof.

In some embodiments, a full length of single-chain variable fragment (scFv) of the murine anti-CD19 binding domain comprises an amino acid sequence of SEQ ID NO: 9, or an amino acid sequence with at least 90% identity thereof.

In some embodiments, the murine anti-CD19 binding domain comprises a nucleic acid sequence encoding the murine anti-CD19 binding domain, wherein the nucleic acid sequence comprises SEQ ID NO: 10, or the nucleic acid sequence with at least 90% identity thereof.

In some embodiments, the CD8 hinge comprises an amino acid sequence of SEQ ID NO: 11, or an amino acid sequence with at least 90% identity thereof. In some embodiments, the CD8 hinge comprises a nucleic acid sequence encoding the CD8 hinge, and the nucleic acid sequence comprises SEQ ID NO: 12, or the nucleic acid sequence having at least 90% identity thereof.

In some embodiments, the 4-1BB comprises an amino acid sequence of SEQ ID NO: 13, or an amino acid sequence with at least 90% identity thereof. In some embodiments, the 4-1BB comprises a nucleic acid sequence encoding the 4-1BB, and the nucleic acid sequence comprises SEQ ID NO: 14, or the nucleic acid sequence with at least 90% identity thereof.

In some embodiments, the CD3 zeta comprises an amino acid sequence of SEQ ID NO: 15, or an amino acid sequence with at least 90% identity thereof. In some embodiments, the CD3 zeta comprises a nucleic acid sequence encoding the CD3 zeta, and the nucleic acid sequence comprises SEQ ID NO: 16, or the nucleic acid sequence with at least 90% identity thereof.

In some embodiments, a full length of the CAR comprises an amino acid sequence of SEQ ID NO: 17, or an amino acid sequence with at least 90% identity thereof. In some embodiments, a full length of the CAR comprises a nucleic acid sequence encoding the CAR, wherein the nucleic acid sequence comprises SEQ ID NO: 18 or the nucleic acid sequence with at least 90% identity thereof.

In some embodiments, a nucleic acid sequence with at least 90% identity to SEQ ID NO: 10, 12, 14, 16, 18 are that the sequence having 90% to 99% identity to SEQ ID NO: 10, 12, 14, 16, and 18, respectively, can also provide the same effect in the present embodiments. For examples, 91%, 92%, 93%, 94%, 95%, 96%, 97%, 98%, or any value between any two of these values.

In some examples, the nucleic acid sequence is selected from a degenerate sequence of SEQ ID NO: 10, 12, 14, 16, and 18. The term "degenerate sequence" refers to that a portion of nucleotides of the nucleic acid sequence in the present disclosure is replaced by other nucleotides. In other words, the degenerate sequence of SEQ ID NO: 10, 12, 14, 16, and 18 refer to that when the sequence length of the SEQ ID NO: 10, 12, 14, 16, and 18 are constant, the oligonucleotide sequence having about 1% to 10% variation can be tolerated.

In some examples, the nucleic acid sequence is selected from a derived sequence of SEQ ID NO: 10, 12, 14, 16, and 18. The term "derived sequence" refers to that 3' end and/or 5' end of the oligonucleotide sequence in the present disclosure is modified, and part or all of the oligonucleotide sequence can be reserved.

The term "homologous" or "identity" refers to the subunit sequence identity between two polymeric molecules, e.g., between two nucleic acid molecules, such as, two DNA molecules or two RNA molecules, or between two polypeptide molecules. When a subunit position in both of the two molecules is occupied by the same monomeric subunit; e.g., if a position in each of two DNA molecules is occupied by adenine, then they are homologous or identical at that position. The homology between two sequences is a direct function of the number of matching or homologous positions; e.g., if half (e.g., five positions in a polymer ten subunits in length) of the positions in two sequences are homologous, the two sequences are 50% homologous; if 90% of the positions (e.g., 9 of 10), are matched or homologous, the two sequences are 90% homologous.

In some embodiments, the present disclosure pertains an isolated polypeptide molecule encoded by the nucleic acid sequence as above mentioned.

In some embodiments, the invention pertains to a vector comprising a nucleic acid sequence encoding a CAR. In one embodiment, the vector is selected from the group consisting of a DNA, a RNA, a plasmid, a lentivirus vector, adenoviral vector, or a retrovirus vector.

In some embodiments, the vector is a lentivirus vector. In one embodiment, the vector further comprises a promoter. In some examples, the promoter is an EF-1 promoter. In some examples, the promoter is an EF-1α promoter. In some examples, the vector is an in vitro transcribed vector.

In some embodiments, a cell comprises the vector as above mentioned. In one embodiment, the cell is a human T cell. In some examples, the T cell is a CD8+ T cell.

A number of examples are provided herein to elaborate the murine anti-CD19 chimeric antigen receptor of the instant disclosure. However, the examples are for demonstration purpose alone, and the instant disclosure is not limited thereto.

### Example

Although a series of operations or steps are used below to describe the method disclosed herein, an order of these operations or steps should not be construed as a limitation to the present invention. For example, some operations or steps may be performed in a different order and/or other steps may be performed at the same time. In addition, all shown operations, steps and/or features are not required to be executed to implement an embodiment of the present invention. In addition, each operation or step described herein may include a plurality of sub-steps or actions.

### Example 1 Lentivirus production

Lentivirus is a critical material for the production of CAR-T cells. Lentiviruses are used for the transduction of specifically designed CAR plasmid sequences into human T cells, to produce CAR-expressing T cells.

To prepare cells for lentivirus production, adherent 293T cells cultured in flasks were detached using trypsin. Following termination of trypsinization by culture medium supplemented with FBS, 293T cells were collected by centrifugation and resuspended in 293T culture medium (10% FBS in DMEM, 2 mM glutamine, and 1 mM pyruvate). The number and viability of 293T cells were determined by staining cells using Acridine Orange/Propidium Iodide and automated cell counting with LUNA-FL^{™} Automated Fluorescence Cell Counter (abbreviated as AO/PI and cell counter). For producing lentivirus using 293T cells in T875 flask, 1.5 × 10⁷ cells were resuspended in 200 mL culture medium, mixed well, then pipetted into a T875 flask. The cell containing T875 were then placed in a humidified incubator at 37°C/ 5% CO₂ for cell culturing.

Following 3 days of culture, lentivirus packaging was initiated by incubating 293T cells with medium containing transfection reagent PolyJet^{™}, and four individual DNA plasmids comprised of GAG-Pol, Rev, VSV-G, CD19 transgene (including murine anti-CD19 binding domain, CD8 hinge, 4-1BB, and CD3 zeta as mentioned above), and with or without a fifth DNA plasmid of TAT. Construction of DNA plasmids were mainly synthesized via outsourcing and modified by molecular cloning techniques, and transfection procedures using transfection reagent PolyJet^{™} were conducted according to manufacturer protocol. The original culture medium for 293T was aspirated and replaced with 108 mL of serum-free DMEM, which included 8 mL of PolyJet/DNA pre-mixture. The T875s were then placed in a humidified incubator at 37°C/ 5% CO₂ for cell transfection.

After 12-18 hours of transfection, the cell culture was rinsed with PBS, then supplemented with 100 mL of OPTI-MEM medium (Opti-MEM, 2 mM glutamine, 1 mM pyruvate, 0.25% albumin, and 20 mM HEPES) for the collection of lentivirus comprising supernatant. The T875s were placed in a humidified incubator at 37°C/ 5% CO₂ for continuous production and collection of lentivirus. Specifically, lentivirus containing supernatants were collected every 24 hours, and for 1-2 times, and replaced with 100 mL of fresh OPTI-MEM medium following each collection.

After each batch of lentivirus containing supernatant were collected, they were clarified through a 0.45 µm filter cup and temporary stored at 4°C. Lentivirus samples were further purified, buffer exchanged, and concentrated using a tangential flow filtration (TFF) device KrosFlo, in accordance to manufacturer instructions. Using sterile containers, tubing, hollow fiber that are connected in particular orders, and lentivirus samples that were driven by pressure created via a peristaltic pump, lentiviruses were clarified of potential contaminants such as cell and cell debris, exchanged to buffers and concentrated to higher concentrations that were more appropriate for T cell transduction. The TFF-processed lentiviruses were sterilized by passing through a 0.2 µm filter and stored for later usage at -80°C.

CD19 CAR construct comprises a single chain antibody comprising a murine anti-CD19 binding domain, a transmembrane domain, a costimulatory domain, and a primary intracellular signaling domain. The murine anti-CD19 binding domain includes a VH region and a VL region. The VH region includes a CDR H1 (SEQ ID NO: 1), a CDR H2 (SEQ ID NO: 2), and a CDR H3 (SEQ ID NO: 3); and the VL region includes a CDR L1 (SEQ ID NO: 4), a CDR L2 (SEQ ID NO: 5), and a CDR L3 (SEQ ID NO: 6). A full length of VH region comprises SEQ ID NO: 7 and A full length of VL region comprises SEQ ID NO: 8. A full length of scFv of the murine anti-CD19 binding domain comprises an amino acid sequence of SEQ ID NO: 9, and a nucleic acid sequence encoding the murine anti-CD19 binding domain is SEQ ID NO: 10. The transmembrane domain is CD8 hinge, and the CD8 hinge includes an amino acid sequence of SEQ ID NO: 11, and a nucleic acid sequence encoding the CD8 hinge is SEQ ID NO: 12. The costimulatory domain is 4-1BB, and the 4-1BB comprises an amino acid sequence of SEQ ID NO: 13, and a nucleic acid sequence encoding the 4-1BB is SEQ ID NO: 14. The primary intracellular signaling domain is a native intracellular signaling domain of CD3 zeta, and the CD3 zeta includes an amino acid sequence of SEQ ID NO: 15, and a nucleic acid sequence encoding the CD3 zeta is SEQ ID NO: 16. A full length of the CAR comprises an amino acid sequence of SEQ ID NO: 17, and a nucleic acid sequence encoding the CAR is SEQ ID NO: 18.

### Example 2 CAR-T production

Peripheral blood mononuclear cells (PBMCs) were isolated from healthy patient whole blood samples using Ficoll^{®}-Paque Premium density gradient medium and SepMate^{™}, according to procedures of the manufacturer. Following isolation, PBMCs were resuspended in 2% human albumin in PBS at a cell density of 2 × 10⁷ cells/mL to 5 × 10⁷ cells/mL.

To qualify the PBMC, its cell size, viability, and total number were determined by staining cell using AO/PI and cell counter. To quantify the number of T cells, PBMCs were also stained using a DuraClone IM Phenotyping BASIC tube, and analyzed for its %CD3+ by flow cytometry (CytoFLEX).

To further isolate T cells from resuspended PBMC samples and activate them, Dynabeads^{®} magnetic beads were added to the PBMC cell suspension, in amounts based on the total number of CD3+ T cells, targeting specific Dynabeads^{®} to T cell ratio. Mixed Dynabeads^{®} and PBMC samples were rotated slowly, then placed in the magnetic holder to immobilize and select the beads and cells that formed bead-cell complexes. The cell suspension buffer of 2% human albumin in PBS was then substituted with complete X-VIVO^{™} 15 medium for T cell culturing.

The total number of T cells isolated with Dynabeads^{®} was quantified (AO/PI and cell counter) and T cells (bead-cell complexes) were seeded into flasks at cell densities of 1 × 10⁶ cells/mL. The flasks containing T cells would then be placed in a humidified incubator at 37°C/ 5% CO₂ for overnight culturing of T cells.

After overnight culture, lentiviruses, which titers were previously determined, were defrosted and added to the flask containing T cells and mixed with the culture medium by gentle pipetting of at least 20 times. The flasks were returned to the humidified incubator at 37°C/ 5% CO₂ for lentiviral transduction and further expansion of the T/CAR-T cells.

After another 2 days of culture, the Dynabeads^{®} were dissociated from the bead-cell complex by fixing the magnetic beads using a magnetic holder, then rinsing the cells off the magnetic beads with the pre-existing culture medium. In order to ensure more complete dissociation of beads from the cells, and minimal contamination of cell product by the magnetic beads, de-beading procedure was repeated for multiple times. The dissociated CAR-T cells were then checked for characteristics of cell size, viability, and total number using AO/PI staining and a cell counter.

CAR-T cells were further expanded using a G-Rex^{®} 10M rapid expansion cell culture system for 4 days. 3 × 10⁶ CAR-T cells were added to G-Rex and cultured in 100 mL of complete X-VIVO^{™} 15. The G-Rex containing CAR-T cells were placed in a humidified incubator at 37°C/ 5% CO₂ for 4 days.

Following 4 days of G-Rex^{®} culture, the G-Rex^{®} was inverted to mix and expanded CAR-Ts were sampled using a syringe and a three-way valve. The total CAR-T cell number, which is used to calculate the fold of expansion, was quantified using AO/PI staining and a cell counter. To determine the percentage of T cells and more specifically, the percentage of CAR-T cells, the cells were not only stained with DuraClone IM Phenotyping BASIC tube, to analyze for its %CD3+, but also stained with biotinylated α-murine F(ab')2 antibody, to measure its %CAR+, both quantified using flow cytometry (CytoFLEX). The resulting, characterized CAR-T cells were then applied to perform various experiments.

### Example 3 CAR-T activation and cytotoxicity

CD107a degranulation process involves the fusion of the granule membrane with the cytoplasmic membrane of the immune effector cells, resulting in surface exposure of lysosomal associated proteins. These lysosomal associated proteins are typically present on the lipid bilayer surrounding lytic granules, such as CD107a. Therefore, membrane expression of CD107a serves as constitutes a marker of immune cell activation and cytotoxic degranulation.

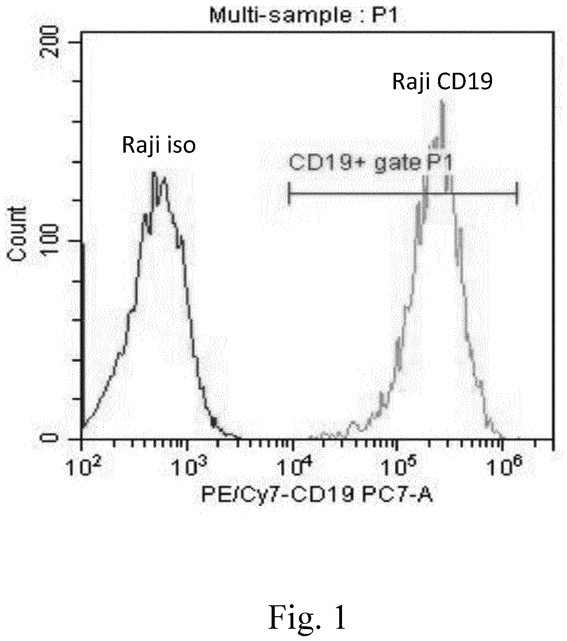

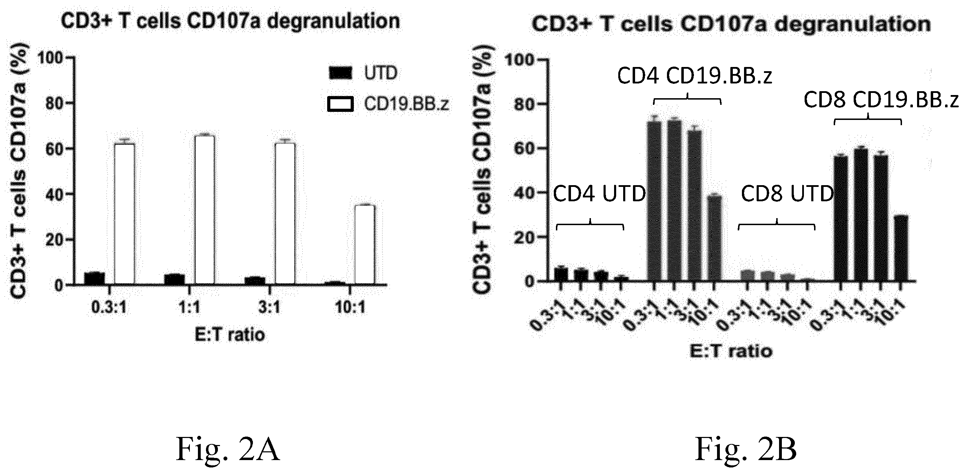

Raji cancer cell line is CD19+, with surface CD19 expression reaching 99.96% under P1 gating using flow cytometry (Fig. 1). When co-culturing Raji cells with untransduced or CAR transduced primary T cells for 5 hours at different effector to target (E:T) ratio (0.3:1, 1:1, 3:1, and 10:1), significant CD107a degranulation was only observed for CAR transduced T cells (Fig. 2A). It seems that CD4+ CAR-T subset had a more substantial degranulation effect than the CD8+ CAR-T cell subset (Fig. 2B).

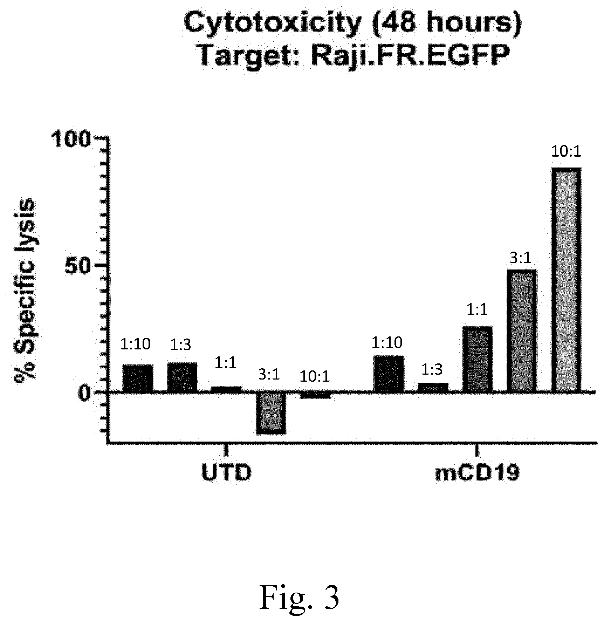

Luciferase based cytotoxicity experiments further showed that more than 20%, 40%, and 80% of the CD19+ Raji cells were lysed upon co culturing with CAR transduced T cells at an E:T ratio of 1:1, 3:1, and 10:1 for 48 hours, respectively (Fig. 3). The cytotoxicity is enhanced at a higher E:T ratio with a significant dose dependent effect.

### Example 4 CAR-T proliferation

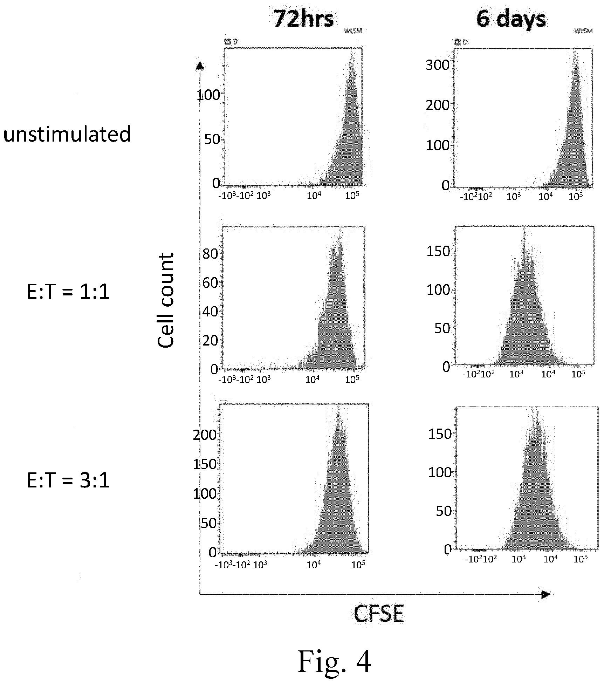

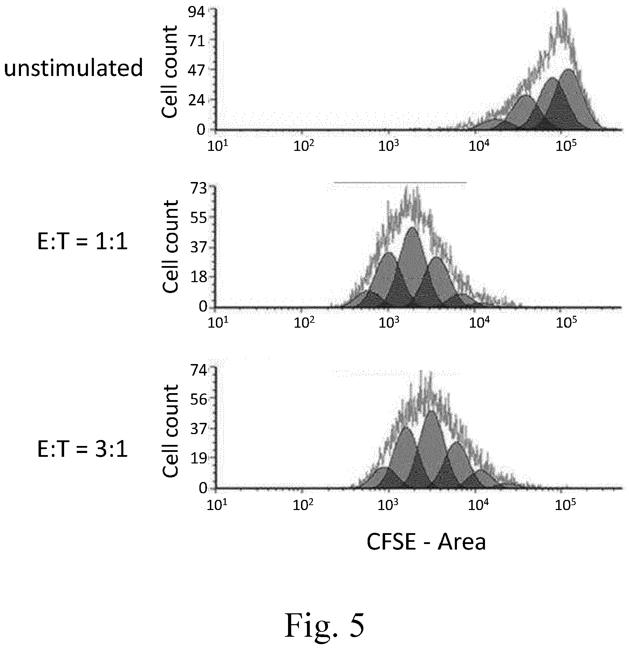

CAR-T cells were stained with carboxyfluorescein succinimidyl ester (CFSE) as a marker in the baseline. The decline of CFSE intensity and proliferation of CAR transduced T cells were observed after exposure to Raji cells at different E:T ratio, starting from 72 hours and lasted up to 6 days. Significant CAR-T proliferation was observed upon Raji exposure (Fig. 4), and the modeling of CAR T cell division (FCS Express^{™} 7, De Novo software, California, the United States) was shown (Fig. 5).

### Example 5 Leukemia xenograft animal model

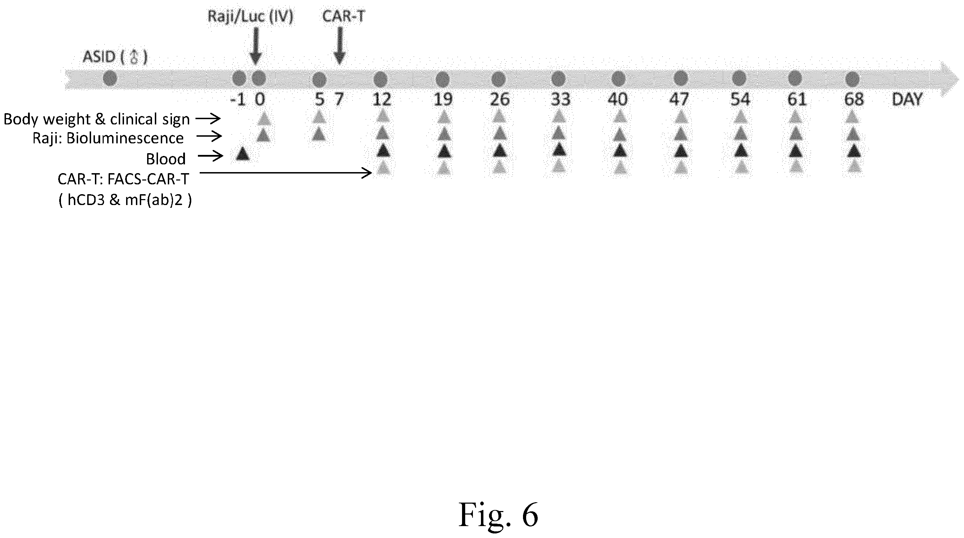

Raji/Luc cells were injected into the tail vein (1 ×10⁶ cells/200 µL/mouse) of 6 to 8 week old male ASID mice (designated as day 0). On day 7 (D7), mice were treated with a single intravenous (i.v.) dose of CAR-T cells. Tumor progression was monitored by bioluminescence imaging (BLI) on scheduled dates. Body weight and clinical sign observation of mice were recorded every week. CAR-T cell persistence was monitored by fluorescence activated cell sorting (FACS) every week. The luciferase activity of organs (heart, liver, spleen, lung, kidney, brain and femur) was measured via BLI (Fig. 6).

Table 1 shows the group allocation and administration. The 1st block factor for grouping Raji/Luc cell injection was the body weight of mice. According to the body weight, mice were assigned to ten levels (from low to high, 4 mice in one level). One mice of each level was allocated to each group randomly. The 2nd block factor for grouping CAR-T cell injection was bioluminescence signal. According to the bioluminescence signal, mice were assigned to ten levels (from low to high, 4 mice in one level). One mice of each level was allocated to each group randomly.

**Table 1**

| Group | Raji/Luc (cells/200 µL/mouse) | CAR-T in saline (cells/500 µL/mouse) | E:T ratio (Effector-to -Target) | Administration Route | Number of Mice |
|---|---|---|---|---|---|
| G1 | 1×10⁶ | Saline | 0 | i.v. | 10 |
| G2 | 1×10⁶ | 1×10⁶ | 1:1 | i.v. | 10 |
| G3 | 1×10⁶ | 3×10⁶ | 3:1 | i.v. | 10 |
| G4 | 1×10⁶ | 1×10⁷ | 10:1 | i.v. | 10 |

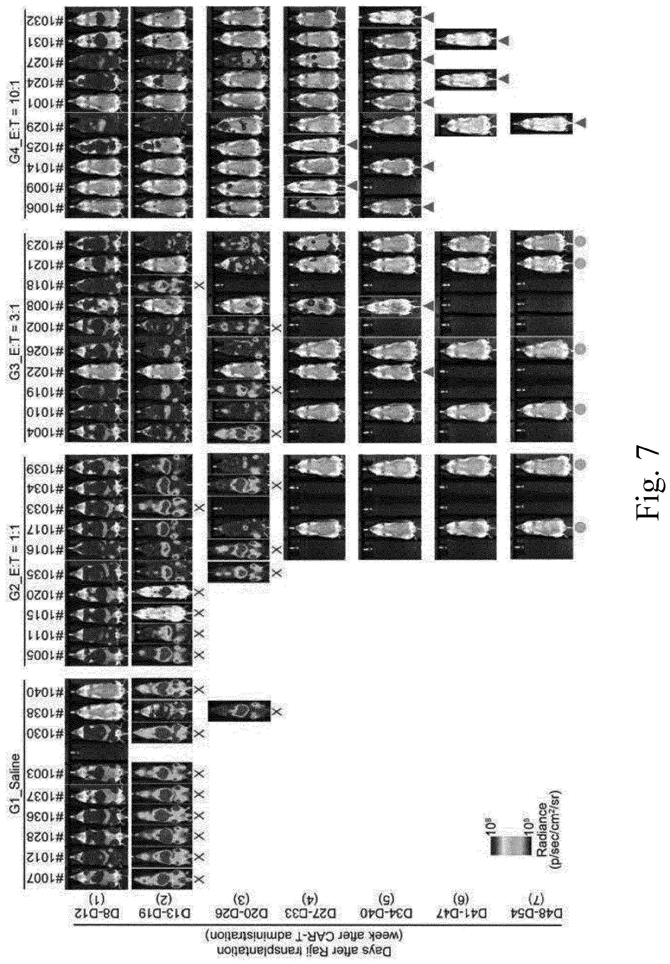

As shown in Fig. 7, bioluminescent images of mice of G1 to G4 were obtained at indicated periods in the same imaging condition. Symbols X, ▲ , and •, indicating humane endpoint on D17 to D25 (with paralysis symptom in majority of mice), humane endpoint on D28 to D56 (with weight loss in majority of mice) and experiment endpoint on D56 (alive), respectively. The mean with SD of bioluminescence intensity in each group (×10³, photons/sec/cm²/sr) is shown in Table 2.

**Table 2**

| Days after Raji transplantation | Group | | | |
|---|---|---|---|---|
| | G1_Saline | G2_E:T=1:1 | G3_E:T=3:1 | G4_E:T=10:1 |
| D0 | 12.8±7.5 (n=9) | 17.2±9.0 (n=10) | 11.7±6.5 (n=10) | 16.5±12.4 (n=10) |
| D5 | 28.2±18.7 (n=9) | 32.2±19.4 (n=10) | 32.4±30.3 (n=10) | 43.6±44.0 (n=10) |
| D12 | 1261.0± 1120.3 (n=9) | 2116.1± 939.5 (n=10) | 1493.4± 1171.0 (n=10) | 1584.2± 2139.7 (n=10) |
| D19 | 43571.5± 25067.4 (n=5) | 15193.4± 10395.7 (n=8) | 5609.8± 6175.3 (n=10) | 1271.1± 2608.0 (n=10) |
| D26 | -(n=0) | 7453.9±2409.3 (n=2) | 6290.8±7291.3 (n=6) | 367.8±844.7 (n=10) |
| D33 | -(n=0) | 8.4±2.9 (n=2) | 245.5±225.6 (n=6) | 72.6±87.7 (n=8) |
| D40 | - | 4.7±0.8 | 21.6±38.8 | 3.0±0.5 |
| | (n=0) | (n=2) | (n=5) | (n=7) |
| D47 | -(n=0) | 3.9±0.2 (n=2) | 3.5±0.4 (n=4) | 3.1 ±0 (n=1) |
| D54 | -(n=0) | 4.6±0.4 (n=2) | 3.9±0.8 (n=4) | -(n=0) |

The result indicates that mice in G1 group with saline were dead after Raji cells injection within second to third weeks. On the contrary, the bioluminescence intensity was decrease at a higher E:T ratio with a significant dose dependent effect in first one to three weeks, and the death from D17 to D25 with paralysis symptom in majority of mice (marked with symbol x in Fig. 7) was due to cancer. It is noticed that the death from D28 to D56 with weight loss in majority of mice (marked with symbol ▲ in Fig. 7) was due to Graft-versus-host disease (GVHD) which was further confirmed by pathological section. Furthermore, as shown in G4 group, the bioluminescence intensity decrease in first to second weeks indicated that the cancer was diminished in early weeks, but the death from D28 to D56 with weight loss in majority of mice was due to GVHD.

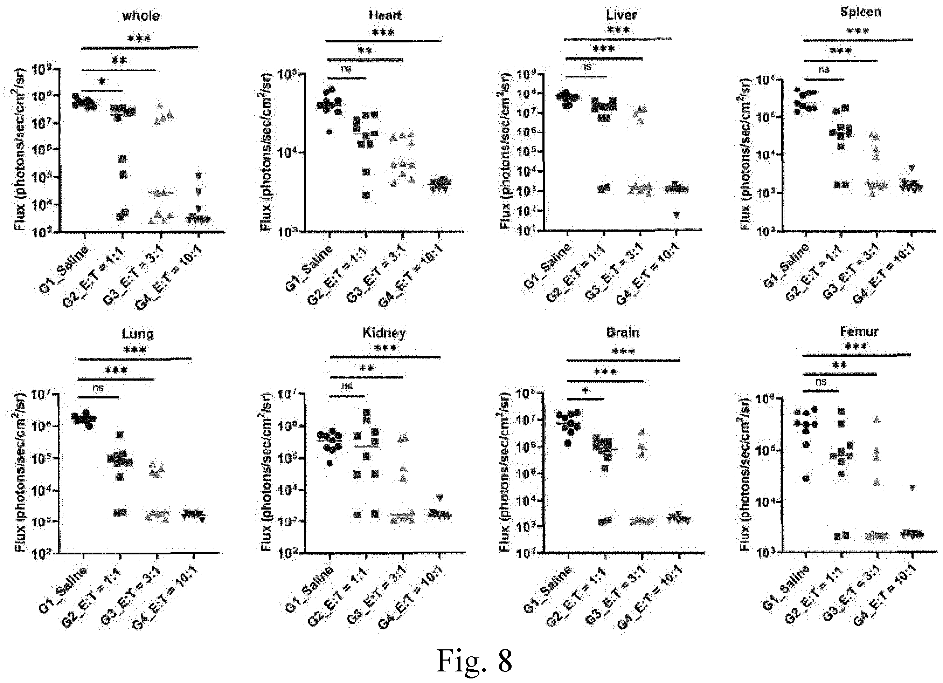

Fig. 8 is in vivo bioluminescence intensity of organs (heart, liver, spleen, lung, kidney, brain and femur) and whole body of Raji/Luc transplanted mice on the day of sacrifice. The mean with SD of bioluminescence intensity of indicated organs on the day of sacrifice in each group (photons/sec/cm²/sr) is shown in Table 3.

**Table 3**

| Group (N) | G1_Saline (9) | G2_E:T=1:1 (10) | G3_E:T=3:1 (10) | G4_E:T=10:1 (10) |
|---|---|---|---|---|
| whole body | 57325396.4± 17979822.9 | 17454301.7± 16103688.3 | 9117295.1± 14242783.0 | 15977.2± 32204.0 |
| heart | 417352± 13602.1 | 17374.35299± 9422.4 | 9733.0± 5187.7 | 3895.8± 450.3 |
| liver | 62148572.4± | 16896901.1 ± | 4563827.9± | 1127.0± |
| | 29879570.9 | 14894036.5 | 6784246.3 | 498.6 |
| spleen | 296214.4± 146780.9 | 53588.3± 56674.6 | 9443.9± 12354.6 | 1761.8± 906.3 |
| lung | 1696795.6± 456721.6 | 114139.3± 154135.9 | 19536.4± 24538.8 | 1436.3± 671.3 |
| kidney | 359111.7± 203896.5 | 582053.9± 865037.5 | 92783.4± 175070.4 | 1893.0± 1173.7 |
| femur | 338462.0± 196689.1 | 136430.8± 176257.8 | 60249.1± 122200.5 | 3714.7± 4848.0 |
| brain | 9389766.3± 6207629.4 | 822901.7± 700304.8 | 617085.6± 1116086.5 | 1979.1± 461.0 |

The result indicates that the bioluminescence intensity represented that the Raji cells were located in whole body such as heart, liver, spleen, lung, kidney, brain and femur. The bioluminescence intensity in all organs was decrease at a higher E:T ratio with a significant dose dependent effect.

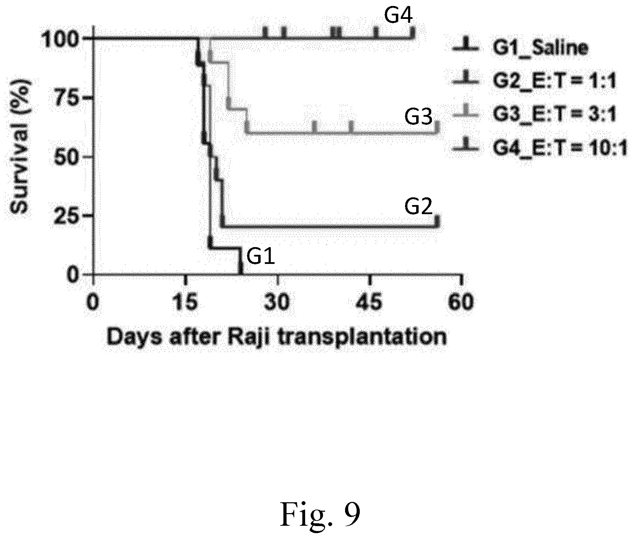

As shown in Fig. 9, Fig. 9 is Kaplan-Meier curve of Raji/Luc cancer model administrated with CAR-T. The mice that died due to GVHD was excluded, so as to analyze the real survival rate after CAR-T administration. In Burkitt's lymphoma-free survival curve, mice in G1 group with saline only were all dead within 24 days after Raji transplantation. On the contrary, G2 group with E:T=1:1 has 25% survival rate, G3 group with E:T=3:1 has 70% survival rate, and surprisingly that G4 group with E:T=10:1 has 100% survival rate within 28 days after Raji transplantation.

## Claims

1. An isolated nucleic acid molecule encoding a chimeric antigen receptor (CAR), wherein the CAR comprises:
a single chain antibody or single chain antibody fragment comprising a murine anti-CD19 binding domain, wherein the murine anti-CD19 binding domain comprises:
a heavy chain variable (VH) region comprising a heavy chain complementarity determining region (CDR H1), a CDR H2, and a CDR H3; and
a light chain variable (VL) region comprising a light chain complementarity determining region 1 (CDR L1), a CDR L2, and a CDR L3,
wherein the CDR H1 comprises SEQ ID NO: 1, the CDR H2 comprises SEQ ID NO: 2, and the CDR H3 comprises SEQ ID NO: 3; and the CDR L1 comprises SEQ ID NO: 4, the CDR L2 comprises SEQ ID NO: 5, and the CDR L3 comprises SEQ ID NO: 6;
a costimulatory domain comprising 4-1BB; and
a primary intracellular signaling domain comprising a native intracellular signaling domain of CD3 zeta.

2. The isolated nucleic acid molecule encoding the CAR of claim 1, wherein the murine anti-CD19 binding domain is a single chain variable fragment (scFv), a di-scFv, a fragment antigen binding (Fab), or F(ab')2.

3. The isolated nucleic acid molecule encoding the CAR of claims 1 or 2, wherein the VH region comprises SEQ ID NO: 7 or a sequence with at least 90% identity thereof; and the VL region comprises SEQ ID NO: 8, or a sequence with at least 90% identity thereof.

4. The isolated nucleic acid molecule encoding the CAR of any one of claims 1 to 3, wherein the murine anti-CD19 binding domain comprises an amino acid sequence of SEQ ID NO: 9, or an amino acid sequence with at least 90% identity thereof.

5. The isolated nucleic acid molecule encoding the CAR of any one of claims 1 to 4, wherein the murine anti-CD19 binding domain comprises a nucleic acid sequence encoding the murine anti-CD19 binding domain, wherein the nucleic acid sequence comprises SEQ ID NO: 10, or the nucleic acid sequence with at least 90% identity thereof.

6. The isolated nucleic acid molecule encoding the CAR of any one of claims 1 to 5, wherein the 4-1BB comprises an amino acid sequence of SEQ ID NO: 13, or an amino acid sequence with at least 90% identity thereof.

7. The isolated nucleic acid molecule encoding the CAR of any one of claims 1 to 6, wherein the 4-1BB comprises a nucleic acid sequence encoding the 4-1BB, and the nucleic acid sequence comprises SEQ ID NO: 14, or the nucleic acid sequence with at least 90% identity thereof.

8. The isolated nucleic acid molecule encoding the CAR of any one of claims 1 to 7, wherein the CD3 zeta comprises an amino acid sequence of SEQ ID NO: 15, or an amino acid sequence with at least 90% identity thereof.

9. The isolated nucleic acid molecule encoding the CAR of any one of claims 1 to 8, wherein the CD3 zeta comprises a nucleic acid sequence encoding the CD3 zeta, and the nucleic acid sequence comprises SEQ ID NO: 16, or the nucleic acid sequence with at least 90% identity thereof.

10. The isolated nucleic acid molecule encoding the CAR of any one of claims 1 to 9, wherein the CAR sequentially comprising the single chain antibody or single chain antibody fragment, the costimulatory domain, and the primary intracellular signaling domain.

11. The isolated nucleic acid molecule encoding the CAR of any one of claims 1 to 10, further comprising a transmembrane domain of CD8 hinge.

12. The isolated nucleic acid molecule encoding the CAR of claim 11, wherein the CD8 hinge comprises an amino acid sequence of SEQ ID NO: 11, or an amino acid sequence with at least 90% identity thereof.

13. The isolated nucleic acid molecule encoding the CAR of any one of claims 1 to 12, wherein the CAR comprises an amino acid sequence of SEQ ID NO: 17, or an amino acid sequence with at least 90% identity thereof.

14. A vector comprising the nucleic acid molecule encoding the CAR of any one of claims 1 to 13.

15. A cell comprising the vector of claim 14, wherein the cell is a human T cell.
